# EUROPÄISCHE PATENTSCHRIFT

(11) **EP 1 899 337 B1**
(45) Veröffentlichungstag und Bekanntmachung des Hinweises auf die Patenterteilung: **12.08.2009**
(21) Anmeldenummer: 06754281.1
(22) Anmeldetag: 10.06.2006
(51) Int. Cl.: C07D 471/08

(54) **VERFAHREN ZUR HERSTELLUNG VON 3,7-DIAZA-BICYCLO[3.3.1]NONAN-VERBINDUNGEN**
PROCESS FOR THE PREPARATION OF 3,7-DIAZABICYCLO[3.3.1]NONANE COMPOUNDS
PROCEDE POUR REALISER DES COMPOSES DE 3,7-DIAZA-BICYCLO[3.3.1]NONANE

(30) Priorität: 15.06.2005 DE 102005027619
(43) Veröffentlichungstag der Anmeldung: 19.03.2008
(73) Patentinhaber: Clariant Produkte (Deutschland) GmbH, 65929 Frankfurt am Main (DE)
(72) Erfinder: DUECKER, Barbara, 55122 Mainz (DE); WESSLING, Michael, 79400 Kandern (DE); NITSCHKE, Nicole, 65760 Eschborn (DE)
(74) Vertreter: Hütter, Klaus
(86) Internationale Anmeldenummer: PCT/EP2006/005582
(87) Internationale Veröffentlichungsnummer: WO 2006/133869

(56) Entgegenhaltungen:
- WO-A1-02/48301
- WO-A1-02/068574
- BORZEL, HEIDI ET AL: "Iron coordination chemistry with tetra-, penta- and hexadentate bispidine-type ligands" INORGANICA CHIMICA ACTA , 337, 407-419 CODEN: ICHAA3; ISSN: 0020-1693, 2002, XP002400781 in der Anmeldung erwähnt
- COMBA, PETER ET AL: "Catalytic aziridination of styrene with copper complexes of substituted 3,7-diazabicyclo[3.3.1]nonanones" EUROPEAN JOURNAL OF INORGANIC CHEMISTRY , (9), 1711-1718 CODEN: EJICFO; ISSN: 1434-1948, 2003, XP002400782
- SIENER, TOM ET AL: "Synthesis and Opioid Receptor Affinity of a Series of 2,4-Diaryl- Substituted 3,7-Diazabicyclononanones" JOURNAL OF MEDICINAL CHEMISTRY , 43(20), 3746-3751 CODEN: JMCMAR; ISSN: 0022-2623, 2000, XP002400783

## Beschreibung

Die Erfindung betrifft ein verbessertes Verfahren zur Herstellung von 3,7-Diazabicyclo[3.3.1]nonan-Verbindungen, das großtechnisch durchführbar ist, reproduzierbar gute Ausbeuten liefert und geringeren Lösemitteleinsatz erfordert.

3,7-Diaza-bicyclo[3.3.1]nonan-Verbindungen der Formel 1 stellen interessante Verbindungen für verschiedene Anwendungen dar. Unter anderem sind sie selbst oder Übergangmetallkomplexe, die Liganden gemäß Formel (I) enthalten, sehr effektive Bleichkatalysatoren 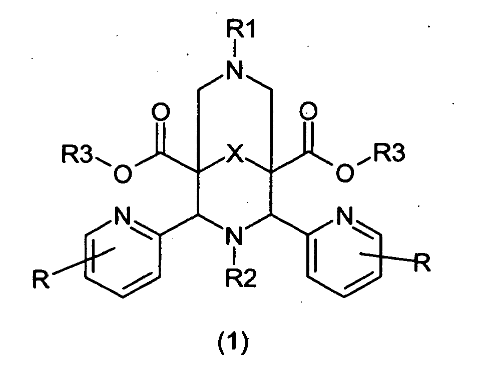 wobei R Wasserstoff, Hydroxyl, C₁-C₄ Alkyl; R¹ C₁-C₄ Alkyl, C₆-C₁₀ Aryl oder Pyridinyl-C₁-C₄-alkyl; R²C₁-C₄ Alkyl, C₆-C₁₀ Aryl; R³ C₁-C₄ Alkyl und X C=O oder C(OH)₂ bedeutet.

Deren Verwendung als Bleichkatalysator in Wasch- und Reinigungsmittel wird u.a. in WO 02/48301, US 2003/0 162 681 und WO 03/104 234 beansprucht.

Die Herstellung dieser Verbindungen erfolgt gemäß den Angaben in Inorg. Chimica Acta, 337 (2002) 407-419 nach folgendem Reaktionsschema:

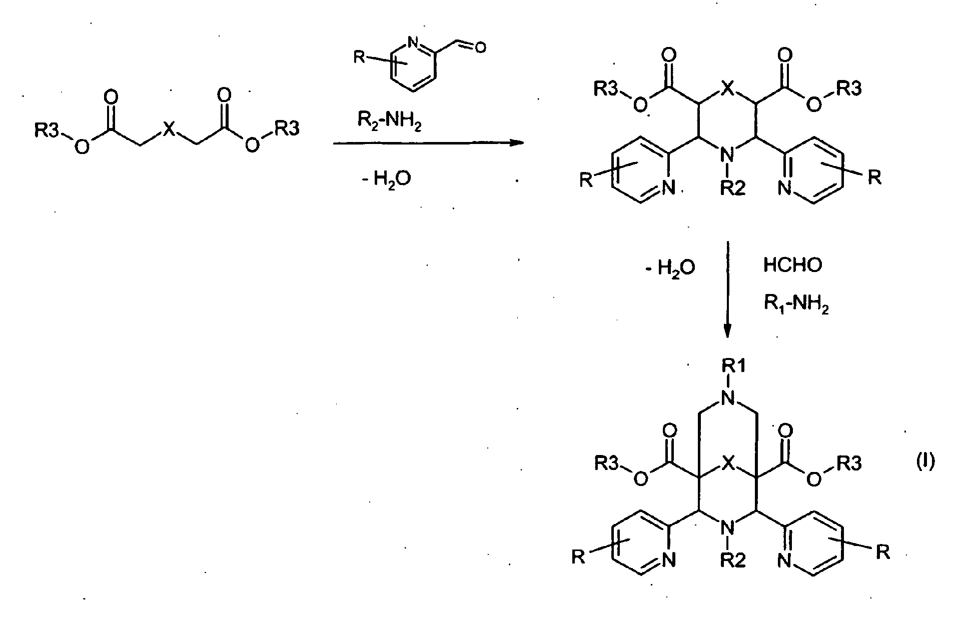

Ausgehend von Dicarbonsäurediester werden in zwei Mannich Kondensationsschritten, jeweils unter Wasserabspaltung Verbindungen gemäß Strukturformel 1 erhalten.
Die Synthese, durchgeführt im Labormaßstab wird im ersten Reaktionsschritt in Methanol durchgeführt; die Isolierung des festen Produktes durch Auskristallisieren bei Temperaturen um ca. 5°C erfordert 1 bis 3 Tage, die Trocknung erfolgt im Vakuum bei 40°C. Im zweiten Reaktionsschritt wird das Produkt des ersten Reaktionsschrittes in Ethanol suspendiert, Amin und Formaldehyd zugegeben und zum Sieden erhitzt. Zur Isolierung des Produktes wird das Ethanol abgezogen, der Rückstand in Ethanol aufgenommen, bei ca. 5°C auskristallisiert und gegebenenfalls in Ethanol umkristallisiert.

Das beschriebene Syntheseverfahren ist im großtechnischen Maßstab nicht durchführbar; der Lösemitteleinsatz ist hoch, die Kristallisation erfordert zu lange Zeiträume, die Isolierung der festen Produkte aus den Reaktionsapparaturen ist problematisch und so technisch nicht durchführbar, die Ausbeuten sind schlecht reproduzierbar.

Aufgabe war deshalb, ein verbessertes Verfahren zur Herstellung von Substanzen der Formel I zu entwickeln, das frei ist von den oben geschilderten Nachteilen.

Gegenstand der Erfindung ist ein Verfahren zur Herstellung von 3,7-Diazabicyclo[3.3.1]nonan-Verbindungen der Formel 1 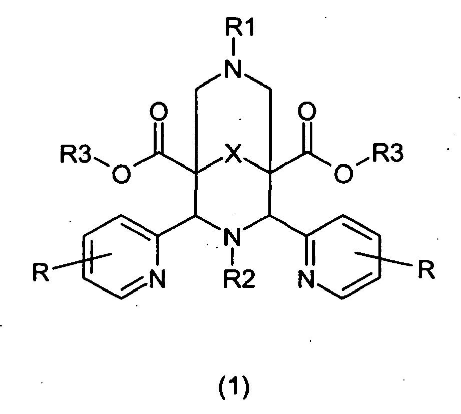 wobei R Wasserstoff, Hydroxyl, C₁-C₄ Alkyl; R¹ C₁-C₄ Alkyl, C₆-C₁₀ Aryl; oder Pyridinyl-C₁-C₄-alkyl; R² C₁-C₄ Alkyl, C₆-C₁₀ Aryl; R³ C₁-C₄ Alkyl und X C=O oder C(OH)₂ bedeutet, durch
(a) Umsetzung eines Dicarbonsäureesters der Formel (2) 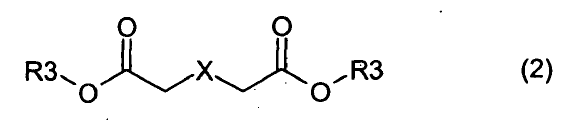 mit einem Pyridinaldehyd der Formel (3) 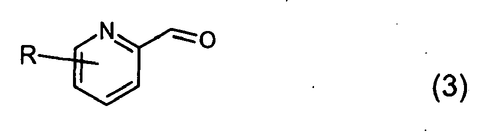 sowie einem Amin der Formel (4)

   R²-NH₂ (4)

   zu einem Piperidon der Formel (5) 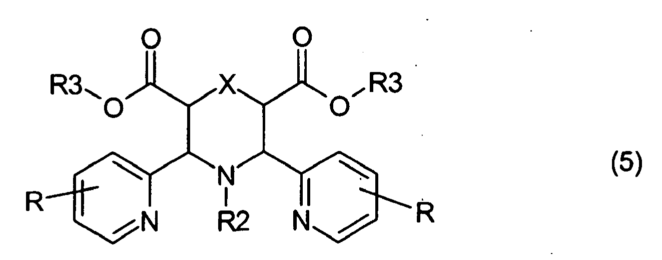 und
(b) Umsetzung der so erhaltenen Piperidonen der Formel (5) mit Formaldehyd und einem Amin der Formel R¹-NH₂, wobei R, R¹, R² und R³ die oben genannten Bedeutungen haben. Dieses Verfahren besteht darin, dass man die Reaktionsstufe (a) in einem C₂-C₄-Alkohol durchführt und die Reaktionsstufe (b) in einem C₃-C₄-Alkohol und das entstandene Reaktionswasser durch azeotrope Destillation entfernt.

Das erfindungsgemäße Verfahren im Einzelnen erfolgt in folgender Weise:

### Reaktionsschritt (a)

Der Dicarbonsäurediester wird in einem C₂-C₄-Alkohol, wie zum Beispiel Ethanol, Propanolen oder Butanolen, bevorzugt in einem verzweigten C₃- oder C₄-Alkoholen vorgelegt und auf 0°C bis 20°C abgekühlt. Zur abgekühlten Mischung wird der betreffende Pyridin-2-aldehyd getropft. Die Menge an Aldehyd beträgt 2,0-2,2 bevorzugt 2,0-2,1 Moläquivalente bezogen auf den Diester. Die Temperatur bei diesem Schritt liegt im Allgemeinen bei 0-20°C, bevorzugt bei 5-15°C, besonders bevorzugt bei 5-10°C. Die Dosierung erfolgt innerhalb von 5-45 Minuten, bevorzugt innerhalb von 10-20 Minuten. Anschließend wird das primäre Amin R₂-NH₂ zugetropft. Die Menge an Amin beträgt 0,9-1,1 bevorzugt 0,95-1,05 Moläquivalente bezogen auf den Diester. Die Temperatur bei dieser Dosierung liegt im Allgemeinen bei 0-20°C, bevorzugt bei 5-15°C, besonders bevorzugt bei 5-10°C. Die Zugabe erfolgt über einen Zeitraum von 30-120 Minuten, bevorzugt innerhalb von 60-90 Minuten. Nach beendeter Zugabe wird die Reaktionsmischung aufgeheizt und im Vakuum durch azeotrope Destillation der Gehalt an Wasser in der Mischung verringert. Die Innentemperatur während der Destillation liegt bei 40-60°C, bevorzugt bei 45-50°C. Das Vakuum wird entsprechend eingestellt. Anschließend wird abgekühlt und nachgerührt, wobei die Temperatur bei 0-20°C liegt. Nach beendeter Nachrührzeit wird das Produkt abfiltriert, mit Lösemittel gewaschen und getrocknet.

Nach dem erfindungsgemäßen Verfahren wird das Zwischenprodukt in Ausbeuten > 80 %, bevorzugt in Ausbeuten von 84-88 % und hoher Reinheit (> 95 % Gehalt nach NMR) erhalten.

### Reaktionsschritt (b)

Das Produkt der ersten Stufe wird wiederum in einem C₃-C₄-Alkohol, wie zum Beispiel in Propanolen oder Butanolen, bevorzugt verzweigten C₃- oder C₄-Alkoholen suspendiert. Der Alkohol in diesem Reaktionsschritt ist vorzugsweise der gleiche Alkohol wie im Reaktionsschritt (a). In beiden Reaktionsschritten können jedoch auch unterschiedliche Alkohole innerhalb der gegebenen Definition genommen werden. Nacheinander werden das Amin R¹-NH₂ und Formalinlösung zugegeben. Die Menge an Amin beträgt 1,2-1,6 bevorzugt 1,4-1,5 Moläquivalente bezogen auf das Zwischenprodukt, die Menge an Formaldehyd beträgt 3,0-4,5 Moläquivalente bezogen auf das Produkt der ersten Stufe. Anschließend wird die Mischung erwärmt und nachgerührt. Die Reaktionszeit beträgt 1-3 Stunden, bevorzugt, 1,5-2 Stunden, die Temperatur liegt bei 50-70°C, bevorzugt bei 55-65°C. Anschließend wird unter Vakuum durch azeotrope Destillation der Gehalt an Wasser im Reaktionsgemisch so weit wie möglich verringert. Nach beendeter Destillation wird zunächst Raumtemperatur dann auf 0-15°C bevorzugt auf 5-10°C abgekühlt und nachgerührt. Dann wird das Produkt abfiltriert, mit frischem Lösemittel gewaschen und getrocknet.

Nach dem erfindungsgemäßen Verfahren wird die Verbindung der Formel I in Ausbeuten > 50 % bevorzugt in Ausbeuten von 55-65 % in hoher Reinheit erhalten (> 98 % Gehalt nach NMR).

Die Herstellung der Verbindungen gemäß Formel 1 nach dem erfindungsgemäßen Verfahren erfordert im Vergleich zum Stand der Technik
- nur ein organisches Lösemittel anstelle von zwei sowie
- eine auf zirka 35 % reduzierte Masse an benötigtem organischem Lösemittel. Das Verfahren ist im Vergleich zum Stand der Technik großtechnisch durchführbar.

Das Verfahren, wie in der Literatur beschrieben, ist großtechnisch in vielen Punkten nicht realisierbar, die Kristallisation erforderte einen hohen zeitlichen Aufwand und ist kaum reproduzierbar. Durch Einführung der Destillation in Stufe 1 und 2 gelang es, eindeutig definierte Parameter für eine reproduzierbare Kristallisation in reduzierter Zeit zu definieren, die Zeitersparnis beträgt zirka 80 %.

Nachfolgende Beispiele sollen die Erfindung näher erläutern ohne sie darauf einzuschränken.

### Beispiel 1

1. Stufe: 12,9 kg Acetondicarbonsäuredimethylester (97 %ig; 72mol) wurden in 52,5 I iso-Butanol gelöst. Die Lösung wurde auf 10°C abgekühlt. Bei dieser Temperatur wurden 15,4 kg Pyridin-2-aldehyd (14,4 mol) zugetropft und 10 Minuten nachgerührt. Zu dieser Mischung wurden anschließend 5,59 kg Methylamin (40 % in Wasser, 72mol) so zugetropft, dass die Temperatur bei gleich bleibender Kühlung gehalten werden konnte. Die Reaktionsmischung wurde 90 Minuten bei 10°C nachgerührt. Nach beendeter Reaktionszeit wurde auf 40-45°C erwärmt und im Vakuum wurden bei 40-45°C Innentemperatur mit Hilfe eines Wasserabscheiders 6,6 kg Wasserphase ausgekreist. Im Anschluss wurde belüftet und auf 5-10°C abgekühlt. Das Produkt wurde abfiltriert und mit 7,5 I iso-Butanol gewaschen. Das feuchte Produkt wurde im Vakuum bei 50°C getrocknet. Es wurden 24,3 kg (88%) 1-Methyl-2,6-Dipyridyl-3,5-di(methoxycarbonyl)-4-piperidon als weiß-beiges Pulver erhalten.
2. Stufe: 23 kg des Produktes der 1. Stufe (60 mol) wurden in 40 I iso-Butanol vorgelegt. Zu dieser Suspension wurden bei Raumtemperatur erst 9,73 kg Aminomethylpyridin (90 mol) dann 19,32 kg Formaldehydlösung (37 %, 24 mol) zugegeben. Nach beendeter Zugabe wurde die Mischung auf 55-60°C erwärmt und 2 Stunden bei dieser Temperatur gerührt. Anschließend wurden bei maximal 60°C Innentemperatur zunächst 16 kg Wasserphase mit Hilfe eines Wasserabscheiders ausgekreist und dann zusätzlich 4 kg iso-Butanolphase abdestilliert. Nach beendeter Destillation wurde belüftet und erst auf Raumtemperatur, dann auf 5-10°C abgekühlt. Nach 2h Rühren bei 5-10°C wurde der Niederschlag abfiltriert, mit 7,5 I iso-Butanol gewaschen und im Vakuum bei 50°C getrocknet. Es wurden 19,8 kg (64%) 2, 4-Di-(pyridyl)-3-methyl-7-(pyridin-2-ylmethyl)-3, 7-diazabicyclo[3.3.1]nonan-9-on-1, 5-dimethyl-dicarboxylat in Form eines fast weißen Pulvers erhalten.
   Ausbeute über 2 Stufen: 56,3 %.

### Beispiel 2

1. Stufe: 44,89 g Acetondicarbonsäuredimethytester (97 %ig, 0,25 mol) wurden in 125 ml iso-Butanol vorgelegt. Die Mischung wurde auf 9°C abgekühlt. Bei dieser Temperatur wurden zunächst 53,55 g Pyridin-2-aldehyd (0,5 mol) zugetropft. Anschließend wurden 27,03 g Aminomethylpyridin (0,25 mol) so zugetropft, dass die Temperatur gehalten werden konnte. Dann wurde die Mischung auf zirka 50°C erwärmt, und im Vakuum bei ca. 50°C Innentemperatur ca. 65 ml Iso-Butanol-Wasser-Gemisch abdestilliert, wobei gleichzeitig ca. 65 ml frisches iso-Butanol zudosiert wurde. Nach beendeter Destillation wurde belüftet und auf 20°C abgekühlt. Das Produkt wurde abfiltriert; mehrfach mit iso-Butanol gewaschen und im Vakuum bei 50°C getrocknet. Es wurden 100,6 g (87,4 %) 1-(Pyridin-2-ylmethyl)-2,6-dipyridyl-3,5-di(methoxycarbonyl)-4-piperidon als beige-weißes Pulver erhalten.
2. Stufe: 69 g des Produktes der 1. Stufe (0,15 mol) wurden in 100 ml Iso-Butanol vorgelegt. Bei Raumtemperatur wurden zunächst 18,0 g Methylamin (40 %ige wässrige Lösung, 0,225 mol), dann 48,7 g Formaldehydlösung (37 % in Wasser; 0,6 mol) zugegeben. Die Mischung wurde auf 60°C erwärmt und 2 h bei dieser Temperatur gerührt. Nach beendeter Reaktionszeit wurden im Vakuum bei 60°C Innentemperatur ca. 200 ml Iso-Butanol-Wasser-Gemisch abdestilliert, wobei gleichzeitig 150 ml frisches iso-Butanol zugetropft wurden. Nach beendeter Destillation wurde belüftet, erst auf Raumtemperatur, dann auf 5°C abgekühlt. Es wurde mehrere Stunden bei 5°C nachgerührt. Anschließend wurde das Produkt abfiltriert, mit iso-Butanol gewaschen und im Vakuum bei 50°C getrocknet. Es wurden 38,7 g (51 %) 2,4-Di-(pyridyl)-3-(pyridin-2-ylmethyl)-7-methyl-3,7-diazabicyclo[3.3.1]nonan-9-on-1,5-dimethyl-dicarboxylat als beige weißes Pulver erhalten.
   Ausbeute über 2 Stufen: 44,6 %.

## Patentansprüche

1. Verfahren zur Herstellung von 3,7-Diaza-bicyclo[3.3.1]nonan-Verbindungen der Formel 1 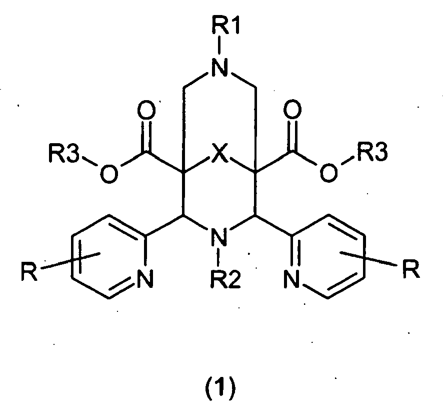 wobei R Wasserstoff, Hydroxyl, C₁-C₄ Alkyl; R¹ C₁-C₄Alkyl, C₆-C₁₀ Aryl, Pyridinyl-C₁-C₄-alkyl; R² C₁-C₄ Alkyl, C₆-C₁₀ Aryl; R³ C₁-C₄ Alkyl und X C=O oder C(OH)₂ bedeutet, durch
(a) Umsetzung eines Dicarbonsäureesters der Formel (2) 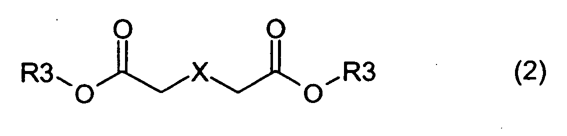 mit einem Pyridinaldehyd der Formel (3) 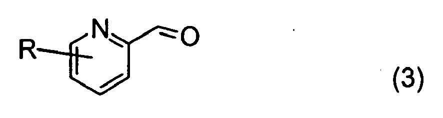 sowie einem Amin der Formel (4)
R²-NH₂ (4)
zu einem Piperidon der Formel (5) 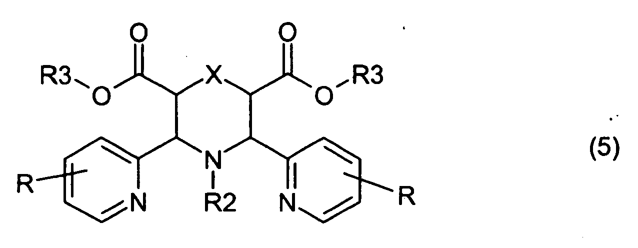 und
(b) Umsetzung der so erhaltenen Piperidonen der Formel (5) mit Formaldehyd und einem Amin der Formel R¹-NH₂, wobei R, R¹, R² und R³ die oben genannten Bedeutungen haben, **dadurch gekennzeichnet, dass** man die Reaktionsstufe (a) in einem C₂-C₄-Alkohol und die Reaktionsstufe (b) in einem C₃-C₄-Alkohol durchführt und das entstandene Reaktionswasser durch azeotrope Destillation entfernt.

2. Verfahren nach Anspruch 1, **dadurch gekennzeichnet, dass** man die Reaktionsstufen (a) und b) in einem verzweigten C₃- oder C₄-Alkohol durchführt.

3. Verfahren nach Anspruch 1, **dadurch gekennzeichnet, dass** man die Reaktionsstufen (a) und (b) in Butanol durchführt.

## Claims

1. A process for preparing 3,7-diazabicyclo[3.3.1]nonane compounds of the formula 1 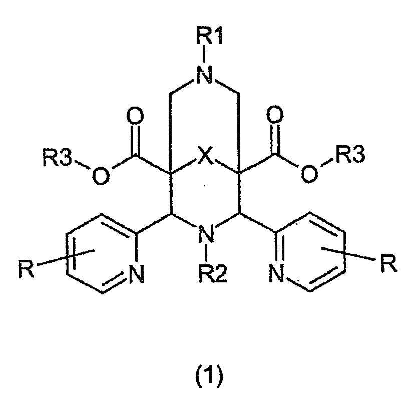 where R is hydrogen, hydroxyl, C₁-C₄ alkyl; R¹ is C₁-C₄ alkyl, C₆-C₁₀ aryl, pyridinyl-C₁-C₄-alkyl; R² is C₁-C₄ alkyl, C₆-C₁₀ aryl; R³ is C₁-C₄ alkyl and X is C=O or C(OH)₂, by
(a) reacting a dicarboxylic ester of the formula (2) 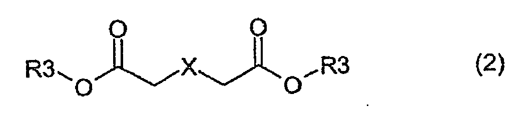 with a pyridinealdehyde of the formula (3) 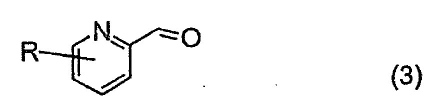 and an amine of the formula (4)
R²-NH₂ (4)
to give a piperidone of the formula (5) 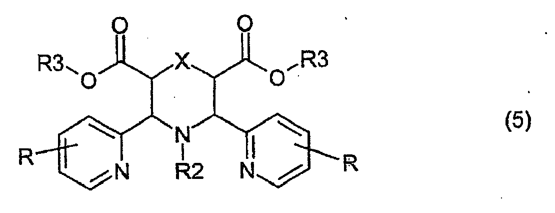 and
(b) reacting the piperidones of the formula (5) thus obtained with formaldehyde and an amine of the formula R¹-NH₂, where R, R¹, R² and R³ are each as defined above, which comprises performing reaction stage (a) in a C₂-C₄-alcohol and reaction stage (b) in a C₃-C₄-alcohol, and removing the water of reaction formed by azeotropic distillation.

2. The process as claimed in claim 1, wherein reaction stages (a) and (b) are performed in a branched C₃- or C₄-alcohol.

3. The process as claimed in claim 1, wherein reaction stages (a) and (b) are performed in butanol.

## Revendications

1. Procédé pour la préparation de composés 3,7-diaza-bicyclo[3.3.1]nonane de formule 1 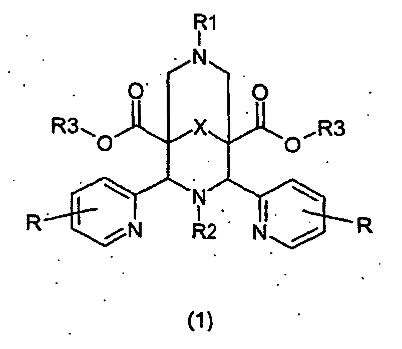 dans laquelle R représente un atome d'hydrogène, un groupe hydroxy, alkyle en C₁-C₄ ; R¹ représente un groupe alkyle en C₁-C₄, aryle en C₆-C₁₀ ou pyridinyl-alkyle (C₁-C₄) ; R² représente un groupe alkyle en C₁-C₄, aryle en C₆-C₁₀ ; R³ représente un groupe alkyle en C₁-C₄ et X représente C=O ou C(OH)₂, par
(a) mise en réaction d'un ester d'acide dicarboxylique de formule (2) 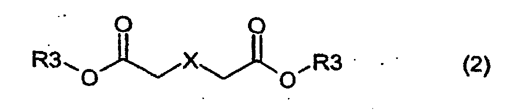 avec un pyridinaldéhyde de formule (3) 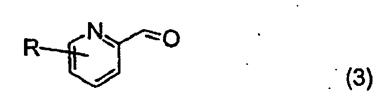 ainsi qu'une amine de formule (4)
R²-NH₂ (4)
pour l'obtention d'une pipéridone de formule (5) 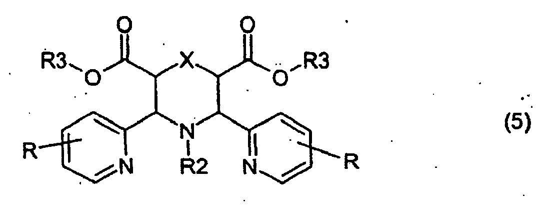 et
b) mise en réaction des pipéridones de formule (5) ainsi obtenues avec du formaldéhyde et une amine de formule R¹-NH₂, R, R¹, R² et R³ ayant les significations données plus haut, **caractérisé en ce qu'**on effectue l'étape de réaction (a) dans un alcool en C₂-C₄ et l'étape de réaction (b) dans un alcool en C₃-C₄ et on élimine par distillation azéotropique l'eau de réaction résultante.

2. Procédé selon la revendication 1, **caractérisé en ce qu'**on effectue les étapes de réaction (a) et (b) dans un alcool en C₃ ou C₄ ramifié.

3. Procédé selon la revendication 1, **caractérisé en ce qu'**on effectue les étapes de réaction (a) et (b) dans du butanol.
